(19) Europäisches Patentamt
European Patent Office
Office européen des brevets

(11) Publication number: **0 257 696 B1**

(12) **EUROPEAN PATENT SPECIFICATION**

(45) Date of publication of the patent specification: **14.11.90**

(51) Int. Cl.[5]: **C07C 2/34,** C07C 2/04

(21) Application number: **87201537.5**

(22) Date of filing: **12.08.87**

(54) **Process for dimerizing alpha-olefins.**

(30) Priority: **15.08.86 US 896700**

(43) Date of publication of application: **02.03.88 Bulletin 88/9**

(45) Publication of the grant of the patent: **14.11.90 Bulletin 90/46**

(84) Designated Contracting States: **BE DE FR GB IT NL**

(56) References cited:
**EP-A- 0 015 603**
**US-A- 4 542 199**
**US-A- 4 658 078**

**CHEMICAL ABSTRACTS, vol. 104, no. 16, 21st April 1986, page 12, abstract no. 130450y, Columbus, Ohio, US; & JP-A-60 217 209 (IDEMITSU KOSAN CO., LTD) 30-10-1985**

(73) Proprietor: **SHELL INTERNATIONALE RESEARCH MAATSCHAPPIJ B.V., Carel van Bylandtlaan 30, NL-2596 HR Den Haag(NL)**

(72) Inventor: **Slaugh, Lynn Henry, 12518 Texas Army Trail, Cypress Texas 77429(US)**
Inventor: **Schoenthal, Galeon Wayne, 2315 Briar Lee, Houston Texas 77077(US)**

(74) Representative: **Aalbers, Onno et al, P.O. Box 302, NL-2501 CH The Hague(NL)**

**EP 0 257 696 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of alpha-olefins having the general formula I

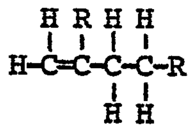

(I)

wherein R represents an alkyl, cycloalkyl or cycloalkenyl group having in the range of from 1 to 30 carbon atoms by dimerization of an alpha-olefin having the general formula II

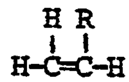

(II)

wherein R has the same meaning as in the general formula I. Products of the general formula I are useful as intermediates in preparing, for example, specialty detergents or lubricant additives.

Metallocene/alumoxane catalysts are known in the art for producing polymers from alpha-olefins. Kaminsky, in Chemical and Engineer News, July 4, 1983, pp 29-30 and in Makromol Chem., Rapid Commun. 4, 417-421 (1983) discloses zirconium and titanium metallocenes in combination with alumoxanes as catalysts for the polymerization of olefins. U.S. patent specification 4,404,344 deals with the use of zirconium metallocenes in combination with alumoxanes as catalysts for the polymerization of ethylene and propylene or copolymerization of ethylene with other alpha-olefins, respectively, with which a controlled molecular weight distribution is obtained. This known process is carried out using an atom ratio aluminium to zirconium between 10:1 and $10^8$:1; in the sole example on polymerization of propylene this atom ratio was $1.5\times10^8$:1.

European patent specification No. 128,045 deals with the use of at least two metallocenes in combination with alumoxane for polymerization of ethylene to products with a broad molecular weight distribution. Metallocenes disclosed are titanocenes, zirconocenes, hafnocenes and vanadocenes.

US 4 542 199 and JP 60 217 209 also disclose processes for the polymerization of olefins which employ catalysts comprising metallocenes and aluminoxanes.

A process of the type described in the beginning has now been found which surprisingly yields dimers with a very high selectivity, little or no polymers being formed. The "selectivity to a certain compound" is defined herein as 100 a:b in which "a" is the amount of starting alpha-olefin of the general formula II that has been converted into that certain compound and "b" is the total amount of starting compound of the general formula II that has been converted.

Accordingly, the invention provides a process for the preparation of alpha-olefins having the general formula I

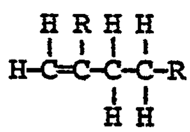

(I)

wherein R represents an alkyl, cycloalkyl or cycloalkenyl group having in the range of from 1 to 30 carbon atoms by dimerization of an alpha-olefin having the general formula II

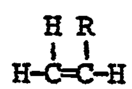

(II)

wherein R has the same meaning as in the general formula I, which process comprises contacting an alpha-olefin having the general formula II at a temperature in the range of from -60°C to 280°C with a catalyst comprising:

a) a metallocene having the general formula III
(cyclopentadienyl)$_2$MY$_2$ (III)
wherein the cyclopentadienyl moieties may optionally be substituted by ($C_1$-$C_5$)alkyl, M represents a zirconium or hafnium atom and each Y individually represents a hydrogen atom, a halogen atom, an alkyl group having in the range of from 1 to 5 carbon atoms or an aryl group having in the range of from 6 to 20 carbon atoms, and

b) an alumoxane, the catalyst having an atom ratio of aluminium to M in the range of from 1 to 100, and recovering an alpha-olefin having the general formula I.

In general, R can not be too bulky or dimerization rates are inhibited. The group R is too bulky if the carbon atom with the free valence is a quaternary carbon atom. It is a routine matter to test a particular olefin with the catalysts used in the instant invention. Mixtures of alpha-olefins can be used as starting materials, resulting in various cross dimerization products. Examples of starting olefins that have been utilized in the instant process are propylene, 1-butene, 1-hexene, 1-octene, 1-eicosene and 4-vinyl-1-cyclohexane. 3,3-Dimethyl-1-butene (neohexene) was found to be unreactive alone but would cross dimerize with 1-octene. Styrene, internal olefins and di-olefins were substantially unreactive in the instant process. Di-olefins, particularly conjugated di-olefins adversely affect the dimerization process.

The aluminoxanes (or alumoxanes) are well known in the art and are polymeric aluminium compounds which can be represented by the general formula $(R\text{-}Al\text{-}O)_n$ which is a cyclic compound and $R(R\text{-}Al\text{-}O)_nAlR_2$, which is a linear compound. In the general formula, R represents an alkyl group having in the range of from 1 to 5 carbon atoms such as, for example, methyl, ethyl, propyl, butyl and pentyl and n is an integer from 1 to about 20. Most preferably, R represents a methyl group. Generally, in the preparation of aluminoxanes from, for example, trimethylaluminium and water, a mixture of the linear and cyclic compounds are obtained.

The aluminoxanes can be prepared in various ways. Preferably, they are prepared by contacting water with a solution of a trialkylaluminium, such as, for example, trimethylaluminium, in a suitable organic solvent such as benzene or an aliphatic hydrocarbon. The solvents that can be used are well-known and include the saturated aliphatic compounds such as butane, pentane, hexane, heptane, octane, isooctane and purified kerosenes; cycloaliphatics such as cyclobutane, cyclopentane, cyclohexane, cycloheptane, methylcycloheptane and the dimethylcyclopentanes; alkenes, such as propylene, butene and 1-octene; aromatic solvents such as benzene, toluene and the xylenes. The major requirements in the selection of a solvent are that it be liquid at reaotion temperatures, that it does not react with water or the aluminoxanes or interfere with the desired dimerization reaction. The solvent must be oxygen-free. Hydroxy, ether, carboxyl, keto, and the like groups adversely affect aluminoxane production. A particularly suitable solvent is the olefin to be dimerized. For example, the alkylaluminium is treated with water in the form of a moist solvent or the alkylaluminium such as trimethylaluminium can be desirably contacted with a hydrated salt such as hydrated copper sulphate or aluminium sulphate.

The aluminoxane can be prepared in the presence of a hydrated copper sulphate. This method comprises treating a dilute solution of trimethylaluminium in, for example, toluene, with copper sulphate represented by the formula $CuSO_4{\cdot}5H_2O$. The ratio of copper sulphate to trimethylaluminium is desirably about 1 mol of copper sulphate for 5 mol of trimethylaluminium. The reaction is evidenced by the evolution of methane.

In general, the molar ratio of alkylaluminium to water will be about 1:1 although variations of this ratio can occur without adversely affecting the aluminoxane product; i.e., the Al/water molar ratio can vary between 0.66:1 to about 2:1, preferably between about 0.75:1 to about 1.25:1. A continuous method for producing aluminoxanes is given in U.S. patent specification 3 300 458, incorporated herein by reference. Another suitable method involves the use of hydrated aluminium salts as given in U.S. patent specification 4 544 762, incorporated herein by reference. Another suitable method is to use water which has been ultrasonically dispersed in a solvent as described in U.S. patent specification No. 4 730 071, filed on even date, incorporated by reference herein or water which has been dispersed using high speed shearing as described in U.S. patent specification No. 4 730 072, filed on even date, incorporated by reference herein.

Preferably each Y in the general formula III individually represents a hydrogen or chlorine atom or a methyl group. It is understood that the two Ys may be the same or different. Bis(cyclopentadienyl)zirconium dichloride is the preferred metallocene.

For the purposes of this invention, included with the definition of the above cyclopentadienyl moiety is the lower alkyl($C_1$-$C_5$)-substituted, preferably the methyl-substituted cyclopentadienyl moiety. Other metallocenes such as those containing titanium, vanadium and niobium have been found not to work adequately in the instant process.

In general terms, the catalyst is prepared by adding the aluminoxane dissolved in an organic solvent, preferably the solvent utilized to prepare the aluminoxane, to a well-stirred suspension of the metallocene in an organic solvent, which can be inert with respect to the catalyst system or, preferably can be the olefin which comprises the feed. When the stirred solution to which the aluminoxane has been added becomes homogeneous, the catalyst has been formed, and if the feed olefin has been used as solvent, the dimerization reaction commences.

Critical to the production of dimers rather than polymers is the selection of the particular metallocene and the particular atom ratio of Al to Zr in the catalyst. The atom ratio of Al to Zr ranges from 1:1 to 100:1 preferably from 1:1 to 50:1. The atom ratio of Al to Zr in the catalyst effects the selectivity and conversion in opposite ways. As the Al/Zr atom ratio increases, the conversion and rate of reaction increases, but the selectivity to the dimer falls off. Also, as the number of carbon atoms per molecule of the alpha-olefin of the general formula II is increased, the Al/Zr atom ratio also must increase to provide equivalent results. Thus, a given Al/Zr atom ratio may be optimum for a given olefin feed, but not for a different olefin feed.

The dimerization reaction is carried out in a conventional fashion. It may be carried out continuously in a stirred tank reactor wherein olefin and catalyst are added continuously to a stirred tank and reactant, product and catalyst are removed from the stirred tank with the product separated and the catalyst and used reactant recycled back to the stirred tank. Alternatively, the reaction may be carried out in a batch reactor, wherein the catalyst, or the catalyst precursors, and reactant olefin are charged to an autoclave, and after being reacted for an appropriate time, product is separated from the reaction mixture by conventional means, such as distillation. The dimerization is preferably carried out at a temperature in the range of 0 °C to 150 °C. Pressures are not critical and can range from about 1 to about 506 bar or higher.

The following Examples further illustrate the invention.

Example 1

A typical catalyst was prepared in a dry box by adding 4.6 g of a solution of 25% by weight of trimethylaluminium (16 mmol) in toluene to a magnetically stirred bottle containing 1.0 g copper sulphate pentahydrate (containing 16 mmol water) in 20 ml toluene. After 15 min the gas evolution slowed and slow stirring was started. At 1.5 h the material was heated to 60 °C and cooled. The liquid was withdrawn and added to a 100 ml autoclave containing 0.29 g (1 mmol) bis(cyclopentadienyl)zirconium dichloride ($Cp_2ZrCl_2$). Propylene (25 g, 0.6 mol) was charged to the autoclave, and the vessel heated at 40-46 °C for 3 h. The pressure rose to a maximum of 13.8 bar and dropped to a final pressure of 3.8 bar. The reaction product was removed and analyzed.

Conversion of the propylene was greater than 95%.

The selectivities to $C_6$-olefins and to $C_9$-olefins were 95.8 and 4.2%, respectively.

NMR analysis of the $C_6$-hydrocarbons showed the following composition:

| Compound | % by weight |
|---|---|
| 2-methyl-1-pentene | 95.8 |
| 2,3-dimethyl-1-butene | 1.8 |
| 2-methylpentane | 2.3 |
| 2,3-dimethylbutane | 0.1 |

It can be seen that the $C_6$-hydrocarbons had a very high content of 2-methyl-1-pentene, being an olefin of the general formula I.

Example 2

A catalyst was prepared as described in example 1 except that the quantities were cut in half. The catalyst was added to 1-octene (200 ml, 1.23 mol) in a 500 ml round bottomed flask fitted with a magnetic stirrer, a thermometer and a nitrogen purged stopcock for sample withdrawal. The flask was heated to about 33-40 °C. After 24 h the conversion of 1-octene was 94.8%, with a selectivity to dimer of 92.6% and to trimer of 3.0%. NMR showed the $C_{16}$ consisted of more than 96% by weight of 2-hexyl-1-decene.

Examples 3-7 and Comparative Experiments A-C

Catalysts were prepared similar to Example 1 with varying Al:Zr ratios. These catalysts were prepared from methylaluminoxane (from trimethylaluminium) and bis(cyclopentadienyl)zirconium dichloride and were used to dimerize propylene. The dimerization was carried out in a 100 ml autoclave, using 21-23 g propylene, a reaction temperature of 20-33 °C and autogenous pressure. A reaction time of 1 h was used. The results presented in Table I hereinafter show that only at an atom ratio Al to Zr below 100 a high selectivity to dimers ($C_6$) is obtained.

TABLE 1

| Example | Comparative Experiment | Mmol $Cp_2ZrCl_2$ | Al:Zr atom ratio | Conversion of propylene, % | Selectivity[1), %, to] $C_6$ | $C_9$ | $C_{12}$ | $C_{15}$ | $C_{18}$ | % wt of propylene to $>C_{18}$, |
|---|---|---|---|---|---|---|---|---|---|---|
| 3 | | 1 | 8 | 9.5 | 97 | 3 | | | | |
| 4 | | 1 | 16 | 50 | 83.7 | 14 | 1.9 | 0.4 | | |
| 5 | | 1 | 24 | 68.5 | 86.1 | 12.7 | 1 | 0.2 | | |
| 6 | | 1 | 32 | 100 | 83.4 | 13.4 | 1.7 | 0.3 | | 5.2 |
| 7 | | 0.5 | 64 | 92 | 66.9 | 23.5 | 4.8 | 0.9 | | 3.9 |
| | A | 0.25 | 128 | 87 | 66.7 | 25.1 | 6.0 | 1.7 | 0.5 | 2.5 |
| | B | 0.076 | 426 | 67 | 43 | 30.6 | 15.3 | 7.5 | 3.8 | |
| | C | 0.055 | 600 | 58.8 | 30.9 | 26.7 | 21.8 | 13.0 | 7.6 | 13.5 (includes polymer) |

1) calculated relative to the total amount of $C_6$-$C_{18}$

Examples 8 and 9 and Comparative Experiment D

These experiments show the effects of the atom ratio Al:Zr on the dimerization of propylene. Example 3 was repeated with a run time of 2 h instead of 1 h using toluene (20 ml), propylene (25-27 g), (cyclopentadiene)$_2$ $ZrCl_2$ and methylaluminoxane, a temperature of 35-47 °C and autogenous pressure. The isomer composition of the dimers was determined. The results observed after a reaction time of 2h and presented in Table II show that only at an atom ratio Al to Zr below 100 a high selectivity to dimers is obtained.

TABLE II

| Example | Comparative Experiment | Al:Zr atom ratio | Conversion of propylene, % | Composition of $C_6$-hydrocarbons, % by weight | | | |
|---|---|---|---|---|---|---|---|
| | | | | 2-methyl-1--pentene | 2,3-dimethyl-1--butene | 2-methyl--pentane | 2,3-dimethyl--butane |
| 8[a)] | | 8:1 | 90 | 95.8 | 1.3 | 1.0 | 0.4 |
| 9[b)] | | 16:1 | 90 | 95.8 | 2.3 | 1.8 | 0.1 |
| | D[c] | 420:1 | 71 | 76 | 13 | 9 | 1 |

a) Oligomer Composition: 97.6%$C_6$, 2.4%$C_9$, 0%$C_{12}$ (weight %).

b) Oligomer Composition: 95.8%$C_6$, 4.2%$C_9$, 0%$C_{12}$ (weight %).

c) Oligomer Composition: 30.3%$C_6$, 22.9%$C_9$, 23.1%$C_{12}$, 12.3%$C_{15}$, 7.8%$C_{18}$, 2.8%$C_{21}$, 0.87%$C_{24}$ (weight %).

Examples 10-13

Catalysts were prepared similar to Example 1 with varying Al:Zr atom ratios. The catalysts were prepared from methylaluminoxane (from trimethylaluminium) and bis(cyclopentadienyl)zirconium dichloride and were used to dimerize 1-octene. The results are shown in Table III.

TABLE III

| Example No. | Al:Zr atom ratio | Reaction temperature °C | Reaction time, h | Conversion of 1-octene[a], % | Selectivity, %, to 2-octene | Selectivity, %, to C16 dimer | Selectivity, %, to C24 trimer | Conversion mol 1-octene per mol catalyst |
|---|---|---|---|---|---|---|---|---|
| 10[b] | 8 | 70 | 19 | 11.2 | 5.8 | 92.7 | 1.6 | 517 |
| 11[c] | 16 | 70 | 1 | 42.2 | 4.7 | 92.4 | 3.1 | 1088 |
| | | | 6 | 77.8 | 5.6 | 91.4 | 3.0 | 1914 |
| 12[d] | 16 | 40 | 6 | 26.4 | 5.0 | 90.2 | 4.8 | |
| | | | 24 | 66.9 | 4.2 | 90.6 | 5.2 | 4110 |
| | | | 118 | 75.2 | 4.3 | 90.8 | 4.9 | 4622 |
| 13[e] | 64 | 40-50 | 2 | 33.8 | 5.1 | 86.2 | 7.7 | |
| | | | 6 | 86.8 | 4.5 | 88.0 | 7.5 | |
| | | | 24 | 97.0 | 4.2 | 88.4 | 7.4 | 5786 |

a) Obtained from Aldrich Chemical Company: 97% 1-octene, 0.07% 2-octene plus 2.93% unspecified.

b) One mmol of I (bis(cyclopentadienyl)zirconium dichloride) and 4760 mmol of octene feed.

c) 0.5 mmol of I and 1230 mmol of octene feed.

d) 0.5 mmol of I and 3075 mmol of octene feed.

e) 0.5 mmol of I and 2979 mmol of octene feed. The amounts of 2-octene produced in 2 h and 24 h were 5.3 g and 12.8 g, respectively.

Examples 14-17

Catalysts were prepared similar to Example 1, from methylaluminoxane (from 4 mmol of trimethylaluminium and 5 mmol of water) and bis(cyclopentadienyl)zirconium dichloride (various amounts) and were used to dimerize 1-hexene (400 mmol). Toluene (30 ml) was used as a solvent and the temperature was 40 °C. The results are presented in Table IV.

TABLE IV

| Example No. | Reaction time h | $Cp_2Zr_2Cl_2$ mmol | Al:Zn atom ratio | Conversion of 1-hexene, % | Selectivity, %, to 2-hexene | $C_7H_{14}$ | dimer | trimer | tetramer |
|---|---|---|---|---|---|---|---|---|---|
| 14 | 1 | 2.0 | 2 | 13.3 | 8.2 | 4.3 | 87.5 | 1.0 | |
| | 2 | | | 34.6 | 5.9 | 2.1 | 88.6 | 3.5 | |
| 15 | 1 | 1.0 | 4 | 63.1 | 4.1 | 1.7 | 91.1 | 3.8 | |
| | 2 | | | 92.5 | 2.5 | 1.2 | 94.5 | 1.9 | |
| 16 | 1 | 0.5 | 8 | 80.8 | 4.7 | 1.8 | 88.3 | 5.2 | |
| 17 | 1 | 0.25 | 16 | 58.6 | 7.0 | 3.2 | 84.6 | 4.7 | 0.6 |
| | 2 | | | 84.2 | 5.5 | 2.2 | 89.2 | 3.0 | 0.2 |

Examples 18-20 and Comparative Experiment E

These experiments illustrate the differences in reactivities as the number of carbon atoms per molecule of the feed increases. The catalysts were prepared from methylaluminoxane (from trimethylaluminium) and bis(cyclopentadienyl)zirconium dichloride and had an atom ratio Al to Zr of 16:1. The feeds were propylene, butene, 3-methyl-1-butene and 3,3-dimethyl-1-butene. Toluene (10 ml) was used as a solvent and a reaction temperature of 28-35 °C was used. The results observed after a reaction time of 1.0 h are presented in Table V. They show that 3-methyl-1-butene can be dimerized, the vinyl group thereof being bound to a tertiary carbon atom. 3,3-Dimethyl-1-butene can not be dimerized, the vinyl group thereof being bound to a quaternary carbon atom and the tertiary butyl group to which this vinyl group is bound being too bulky.

TABLE V

| Example No. | Comparative Experiment | Feed | Mmol | Conversion, % |
|---|---|---|---|---|
| 18 | | Propylene | 476 | 49.4 |
| 19 | | 1-butene | 475 | 41.9 |
| 20 | | 3-Methyl-1-butene | 484 | 23 |
| | E | 3,3-Dimethyl-1-butene | 480 | 0 |

Examples 21-23

Catalysts were prepared similar to Example 1 with differing aluminoxane precursors and bis(cyclopentadienyl)zirconium dichloride at a Al:Zr ratio of 16:1. The experiments were carried out in a 100 ml autoclave, using 21-25 g propylene, 16 mmol of $R_3Al$ and 20 mmol of water from $CuSO_4.5H_2O$, 1.0 mmol of (cyclopentadiene)$_2$ZrCl$_2$, a reaction temperature of 35-45 °C and autogenous pressure. The results presented in Table VI show that the highest conversion is obtained with trimethylaluminium.

TABLE VI

| Examples No. | $R_3Al$ used | Reaction time, h | Conversion of propylene, % | Selectivity, % to Dimer $C_6$ | Trimer $C_9$ | Tetramer $C_{12}$ | Pentamer $C_{15}$ | 2-Methyl-1-pentene content of dimeric material, % |
|---|---|---|---|---|---|---|---|---|
| 21 | trimethyl-Al | 2 | 90 | 95.8 | 4.2 | --- | --- | 95.8 |
| 22 | triethyl-Al | 3 | 62 | 93.2 | 5.2 | 1.0 | 0.6 | 95.2 |
| 23 | tri-isobutyl-Al | 6 | 39 | 98.4 | 1.6 | --- | --- | 90.0 |

Example 24

Bis(cyclopentadienyl)zirconium dimethyl (0.5 mmol), aluminoxane (4 mmol) and 1-octene (50 ml) were heated in the stirred round bottomed flask at 40 °C for 1 h. Analysis of the reaction product showed a 19.1% conversion of 1-octene, with a selectivity to octene dimer of 72.2% and to octene trimer of 19.7%.

Example 25

Bis(cyclopentadienyl)zirconium hydrogen chloride (1 mmol), aluminoxane (8 mmol) and 1-butene (0.47 mol) were heated in an autoclave at 70 °C for 1 h. Analysis of the reaction product showed a 20% conversion of 1-butene, with a selectivity to dimer of 96% and to trimer of 4%.

Example 26

Bis(cyclopentadienyl)zirconium hydrogen chloride (1 mmol), aluminoxane (4 mmol) and 1-octene (50 ml) were heated in an autoclave at 40 °C for 1 h. Analysis of the reaction product showed an 8% conversion of the 1-octene, with a selectivity to the dimer of 72% and to the trimer of 1.5%.

Examples 27-33

In these experiments various amounts of water were used to prepare the aluminoxane and the results on the dimerization catalysts were measured.

The catalysts were prepared as follows: 20 ml of dry toluene were placed in a bottle fitted with a nitrogen purge system and the bottle was placed in an ultrasonic bath (Branson). The ultrasonic was started and the designated amount of water was added through a hypodermic syringe. After a five min period of sonification, 4 mmol of trimethylaluminium (as a 25% by weight solution in toluene) was added. After the reaction was completed (as evidenced by termination of gas evolution), 50 ml of 1-octene and 0.5 mmol of bis(cyclopentadienyl)zirconium dichloride was added and the mixtures were heated to 40 °C. After 30 min samples were removed for analysis. The results specified in Table VIII show that the highest conversions of 1-octene are obtained at an Al:water molar ratio between 0.75:1 and about 1.25:1.

TABLE VIII

| Example No. | Water, mmol | Al/Water molar ratio | Conversion of 1-octene, % |
|---|---|---|---|
| 27 | 3.0 | 0.75 | 37 |
| 28 | 3.2 | 0.8 | 57 |
| 29 | 3.6 | 0.9 | 72 |
| 30 | 3.9 | 0.975 | 67 |
| 31 | 4.0 | 1.0 | 49 |
| 32 | 4.2 | 1.05 | 29 |
| 33 | 4.7 | 1.175 | 12 |

## Claims

1. A process for the preparation of alpha-olefins having the general formula I

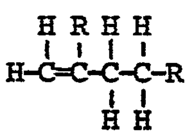

(I)

wherein R represents an alkyl, cycloalkyl or cycloalkenyl group having in the range of from 1 to 30 carbon atoms by dimerization of an alpha-olefin having the general formula II

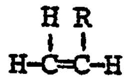

(II)

wherein R has the same meaning as in the general formula I, which process comprises contacting an alpha-olefin having the general formula II at a temperature in the range of from -60°C to 280°C with a catalyst comprising:

a) a metallocene having the general formula III
(cyclopentadienyl)$_2$MY$_2$ (III)
wherein the cyclopentadienyl moieties may optionally be substituted by ($C_1$-$C_5$)alkyl, M represents a zirconium or hafnium atom and each Y individually represents a hydrogen atom, a halogen atom, an alkyl group having in the range of from 1 to 5 carbon atoms or an aryl group having in the range of from 6 to 20 carbon atoms, and

b) an alumoxane, the catalyst having an atom ratio of aluminium to M in the range of from 1 to 100, and recovering an alpha-olefin having the general formula I.

2. A process as claimed in claim 1 wherein each Y in the general formula III individually represents a hydrogen or chlorine atom or a methyl group.

3. A process as claimed in claim 1 or 2 wherein M in the general formula III represents a zirconium atom.

4. A process as claimed in claim 3 wherein the metallocene of the general formula III is bis(cyclopentadienyl)zirconium dichloride.

5. A process as claimed in any one of the preceding claims wherein the atom ratio of aluminium to M is in the range of from 1 to 50.

6. A process as claimed in any one of the preceding claims which is carried out at a temperature in the range of from 0 °C to 150 °C.

**Revendications**

1. Un procédé pour la préparation d'alpha-oléfines ayant la formule générale I

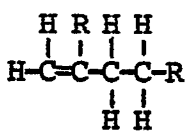

(I)

où R représente un groupe alcoyle, cycloalcoyle ou cycloalcényle ayant de 1 à 30 atomes de carbone par dimérisation d'une alpha-oléfine ayant la formule générale II

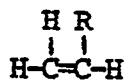

(II)

où R a la même signification que dans la formule générale I, procédé qui comprend la mise en contact d'une alphaoléfine ayant la formule générale II à une température comprise entre -60°C et 280°C avec un catalyseur comprenant:

a) un métallocène ayant la formule générale III
(cyclopentadiényl)$_2$MY$_2$ (III)
où les portions cyclopentadiényle peuvent éventuellement être substituées par des groupes alcoyle de $C_1$ à $C_5$, M représente un atome de zirconium ou de hafnium et chaque Y individuellement représente un atome d'hydrogène, un atome d'halogène, un groupe alcoyle ayant de 1 à 5 atomes de carbone ou un groupe aryle ayant de 6 à 20 atomes de carbone, et

b) un alumoxane, le catalyseur ayant un rapport atomique de l'aluminium à M compris entre 1 et 100, et le recueil d'une alpha-oléfine ayant la formule générale I.

2. Un procédé selon la revendication 1, dans lequel chaque Y dans la formule générale III représente individuellement un atome d'hydrogène ou de chlore ou un groupe méthyle.

3. Un procédé selon la revendication 1 ou 2, dans lequel M dans la formule générale III représente un atome de zirconium.

4. Un procédé selon la revendication 3, dans lequel le métallocène de la formule génerale III est du dichlorure de bis(cyclopentadiényl)zirconium.

5. Un procédé selon l'une quelconque des revendications précédentes, dans lequel le rapport atomique de l'aluminium à M est compris entre 1 et 50.

6. Un procédé selon l'une quelconque des revendications précédentes, qui est mis en oeuvre à une température comprise entre O°C et 150°C.

**Patentansprüche**

1. Verfahren zur Herstellung von α-Olefinen der allgemeinen Formel I

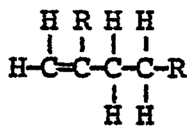

(I)

in der R eine Alkyl-, Cycloalkyl- oder Cycloalkenylgruppe mit 1 bis 30 Kohlenstoffatomen bedeutet, durch Dimerisieren eines α-Olefins der allgemeinen Formel II

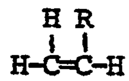

(II)

in der R die gleiche Bedeutung hat wie in der allgemeinen Formel I, dadurch gekennzeichnet, daß man ein α-Olefin der allgemeinen Formel II bei einer Temperatur im Bereich von -60°C bis 280°C mit einem Katalysator in Berührung bringt, der

a) ein Metallocen der allgemeinen Formel III

$(Cyclopentadienyl)_2MY_2$ (III)

worin die Cyclopentadienylgruppen gegebenenfalls durch ($C_1$- bis $C_5$-)Alkyl substituiert sein können, M ein Zirkonium- oder Hafniumatom bedeutet und jedes Y einzeln für ein Wasserstoffatom, ein Halogenatom, eine Alkylgruppe mit 1 bis 5 Kohlenstoffatomen oder eine Arylgruppe mit 6 bis 20 Kohlenstoffatomen steht, und

b) ein Alumoxan umfaßt, wobei der Katalysator ein Atomverhältnis von Aluminium zu M im Bereich von 1 bis 100 aufweist, und daß man ein α-Olefin der allgemeinen Formel I isoliert.

2. Verfahren nach Anspruch 1, dadurch gekennzeichnet, daß jedes Y in der allgemeinen Formel III einzeln für ein Wasserstoff- oder Chloratom oder für eine Methylgruppe steht.

3. Verfahren nach Anspruch 1 oder 2, dadurch gekennzeichnet, daß M in der allgemeinen Formel III für ein Zirkoniumatom steht.

4. Verfahren nach Anspruch 3, dadurch gekennzeichnet, daß das Metallocen der allgemeinen Formel III Bis(cyclopentadienyl)zirkoniumdichlorid ist.

5. Verfahren nach einem der vorangehenden Ansprüche, dadurch gekennzeichnet, daß das Atomverhältnis von Aluminium zu M im Bereich von 1 bis 50 liegt.

6. Verfahren nach einem der vorangehenden Ansprüche, dadurch gekennzeichnet, daß es bei einer Temperatur im Bereich von 0°C bis 150°C durchgeführt wird.